# EUROPEAN PATENT APPLICATION

(11) **EP 3 778 894 A1**
(43) Date of publication of application: **17.02.2021**
(21) Application number: 19785629.7
(22) Date of filing: 09.04.2019
(51) Int. Cl.: C12N 15/113, A01H 1/00, A01H 5/00, C12N 5/10, C12N 15/82, C12P 21/02

(54) **DNA MOLECULE CODING FOR 5' UTR ENABLING HIGH RECOMBINANT PROTEIN EXPRESSION IN MONOCOTYLEDONS**

(30) Priority: 13.04.2018 JP 2018077249
(71) Applicant: National University corporation Nara Institute of Science and Technology, Ikoma-shi Nara 630-0192 (JP)
(72) Inventor: KATO, Ko, Ikoma-shi, Nara 630-0192 (JP)
(74) Representative: Müller-Boré & Partner Patentanwälte PartG mbB
(86) International application number: PCT/JP2019/015506
(87) International publication number: WO 2019/198724

(57) **Abstract**

The purpose of the present invention is to provide technology for identifying a 5' UTR having excellent translation ability while taking into account 5' UTR variants in monocotyledons, providing a DNA molecule that codes for the 5' UTR, and efficiently producing a recombinant protein in the monocotyledons. It is possible to efficiently produce a recombinant protein in a monocotyledon by using a nucleic acid structure in a polynucleotide that codes for a foreign protein, said structure comprising a base sequence among the base sequences shown in sequences 1-4 and linking to a 5' UTR or a variant thereof.

SEQUENCE LISTING

<110>  Nara Institute of Science and Technology

<120>  DNA Molecule Encoding 5 'UTR Enabling High Expression of
       Recombinant Protein in Monocotyledonous Plants

<130>  19007WO

<150>  JP2018-077249
<151>  2018-04-13

<160>  4

<170>  PatentIn version 3.5

<210>  1
<211>  68
<212>  DNA
<213>  Oryza sativa

<400>  1
acaaggcttc ttccacatct ccacccaaat tccaaatctc tcggtgatcg gagcggttag      60

ctgcggcg                                                                68


<210>  2
<211>  129
<212>  DNA
<213>  Oryza sativa

<400>  2
gaacacacaa cccagctata gccacctaca tctgctgatc gacatcgatc gatctctact      60

actccagtag ctagcaagaa ttgaatctag gtgaagtgag agtgtgagag tacataattg     120

gcgccatcc                                                              129


<210>  3
<211>  94
<212>  DNA
<213>  Oryza sativa

<400>  3
accaagagcc acagcgattt cagatctccc caagtagtag acttctctct tcttccatct      60

cggattcgtc tcggtctcgt ctcggtctcc ggcg                                   94


<210>  4
<211>  113
<212>  DNA
<213>  Oryza sativa

<400>  4
cagaaaaacc gcatcccgca actccaccgc tcccaatctc gatcgaaacg cgagggtttc      60

gcatctccgc cgacgcctag aggaagaagg gatccgccgc cggtagccaa gcc            113

## Description

### TECHNICAL FIELD

The present invention relates to a DNA molecule encoding a 5'UTR that enables high expression of a recombinant protein in a monocotyledon. The present invention also relates to a nucleic acid construct including a polynucleotide encoding a protein; and the DNA molecule linked to the polynucleotide encoding a protein, an expression vector including the nucleic acid construct, a transformant including the expression vector, and a method for producing a recombinant protein using the transformant.

### BACKGROUND ART

Conventionally, a technique for introducing a foreign gene into a plant has been established, a high expression system thereof has also been constructed, and a recombinant protein has been actively produced using a plant. Processes of gene expression in a plant are roughly divided into a transcription process and a translation process, and it is known that the initiation reaction of translation is the rate-limiting factor for protein production (Non-Patent Document 1). The translation process is initiated by binding of a translation initiation factor to the Cap structure located at the 5'-terminal of a mRNA and recruitment of the 40S subunit of a ribosome to the 5'untranslated region (5'UTR). Because the recruitment efficiency of a ribosome to a mRNA greatly affects translation efficiency, the scaffold 5'UTR is a very important factor that determines the translation efficiency of mRNA.

Plants are roughly classified into monocotyledons and dicotyledons. Among them, an expression vector in which the 5'UTR sequence of alchol dehydrogenase (ADH) gene (NtADH 5'UTR, AtADH 5'UTR) isolated from tobacco and Arabidopsis thaliana, dicotyledons is incorporated exhibits very high translation capability. A transient expression experiment in which protoplasts of Arabidopsis thaliana and tobacco were used reported that the expression level was improved by as much as 87 to 150 times at the translation stage compared to the commercially available pBI221 vector (Non-Patent Document 2). It is also reported that the 5'UTR sequence of Atlg20440 (AtCOR47) having high translation capability even under various circumstances which has been found from multiple genome-wide studies exhibits its capability in plant bodies of Arabidopsis thaliana and tobacco, avoids the suppression of translation by growth and development of plant bodies and heat stress, and contributes to constant and active translation (Non-Patent Document 3). Thus, for dicotyledons such as Arabidopsis thaliana and tobacco, the 5'UTR sequence having high translation capability has been obtained.

Meanwhile, it has been reported that unlike in dicotyledons, in monocotyledons, even the 5'UTR sequence having high translation capability that has been obtained as described above does not exhibit its translation capability. Specifically, it has been reported that though the AtADH 5'UTR isolated from a dicotyledon exhibits an expression level improving effect in a dicotyledon, the expression level improving effect in Rice cultured cells is only about twice, and even when the ADH 5'UTR sequence isolated from rice (OsADH 5'UTR) was used, the expression level improving effect in Rice cultured cells was at most 9 times (Non-Patent Document 2).

### PRIOR ART DOCUMENTS

### NON-PATENT DOCUMENTS

Non-Patent Document 1: Gebauer, F. and Hentze, M.W. (2004). Molecular mechanisms of translational control, Nat. Rev. Mol. Cell Biol., 5: 827-835
Non-Patent Document 2: Sugio, T., Satoh, J., Matsuura, H., Shinmyo, A., Kato, K. (2008). The 5'-untranslated region of the Oryza sativa alcohol dehydrogenase gene functions as a translational enhancer in monocotyledonous plant cells. J. Biosci. Bioeng. 105: 300-302.
Non-Patent Document3: Yamasaki, S., Sanada, Y., Imase, R., Matsuura, H., Ueno, D., Demura, T., Kato, K. (2018). Arabidopsis thaliana cold-regulated 47 gene 5'-untranslated region enables stable high-level expression of transgenes. J. Biosci. Bioeng. 125: 124-130.

### SUMMARY OF THE INVENTION

### PROBLEMS TO BE SOLVED BY THE INVENTION

Monocotyledons of Poaceae represented by rice, wheat, and corn have a wide variety of uses such as a staple food for human beings, feed for poultry and livestock, and raw materials for bioethanol, and can be said to be the most produced plant species. Thus, obtainment of the 5'UTR sequence having high translation capability that contributes to high expression of a transgene in a monocotyledon is presumably very important.

The present inventor has focused on the number of the data and the accuracy of the data sets used for the analysis as a factor of past failure of the obtainment of a 5'UTR sequence having high translation efficiency in in a monocotyledon. The conventional methodology of 5'UTR search is one in which the translation state of rice endogenous mRNAs is comprehensively evaluated by polysome/microarray analysis and a 5'UTR of mRNAs having a good translation state is isolated. In the methodology, for the evaluation of the translation state, an analysis is performed on a gene-by-gene basis using a microarray, and for the identification of the 5'UTR sequence, the sequence of the full-length cDNA library registered in the database is used.

However, originally, mRNAs derived from one gene have multiple 5'UTR variants each starting from a different transcription start site (TSS). Then, the 5'UTR of a mRNA evaluated to have a good translation state by the conventional 5'UTR search methodology is one evaluated as a set of variants. Thus, conventionally, the 5'UTR of a mRNA (a set of variants) evaluated to have a good translation state is merely isolated based on the information registered in the database. That is, conventionally, the translation state of a 5'UTR is not evaluated in consideration of variants for each TSS. Thus, there is a problem in the number of data and accuracy, and a 5'UTR having high translation capability in a monocotyledon has not been found.

Thus, it is an object of the present invention to provide technology for identifying a 5'UTR really having high translation capability in consideration of 5'UTR variants in a monocotyledon, and efficiently producing a DNA molecule encoding the 5'UTR and a recombinant protein in a monocotyledon.

### MEANS FOR SOLVING THE PROBLEM

The present inventor combined polysome analysis with Cap Analysis of Gene Expression (CAGE) analysis to analyze the translation state of intracellular total mRNA species under normal conditions and heat treatment conditions for rice cultured cells, monocotyledon cells, ranked mRNAs having a good translation state and being actively translated under both conditions, and constructed an expression vector in which a 5'UTR of the mRNA having a high ranking is each linked to a reporter gene to study the translation capability in rice. As a result, a 5'UTR that enables high expression of a heterologous protein even in a monocotyledon was obtained. Its translation capability surpassed the translation capability of OsADH 5'UTR which has been reported to have an expression level improving effect in a monocotyledon. Further, the obtained 5'UTR exhibited high translation capability even when the reporter gene was changed or even when it was applied to rye, another monocotyledon. Further studies based on such findings have been made and thereby the present invention has been completed.

That is, the present invention provides the invention having the aspects described below.
Item 1. A DNA molecule encoding a 5'UTR, having: a polynucleotide shown in any of (i) to (iii) below:
   (i) a polynucleotide consisting of a base sequence shown in any of SEQ ID NOs: 1 to 4,
   (ii) a polynucleotide that consists of a base sequence modified from the base sequence shown in any of SEQ ID NOs: 1 to 4 by substitution, deletion, or addition of one or several bases, and exhibits 5'UTR activity equivalent to that of the polynucleotide consisting of the base sequence shown in any of SEQ ID NOs: 1 to 4, and
   (iii) a polynucleotide that hybridizes under a stringent condition with a DNA fragment consisting of a base sequence complementary to the base sequence shown in any of SEQ ID NOs: 1 to 4, and exhibits 5'UTR activity equivalent to that of the polynucleotide consisting of the base sequence shown in any of SEQ ID NOs: 1 to 4.
Item 2. A nucleic acid construct, including: a polynucleotide encoding a foreign protein; and the DNA molecule according to Item 1 linked to a 5'-terminal side of the polynucleotide encoding a foreign protein.
Item 3. A vector, including:
   the nucleic acid construct according to Item 2.
Item 4. A method for producing a transformant, including the step of:
   introducing the vector according to Item 3 into a monocotyledon or a monocotyledon cell.
Item 5. A transformant obtained by transforming a monocotyledon or a monocotyledon cell with the vector according to Item 3.
Item 6. A method for producing a recombinant protein, including the step of:
   culturing or cultivating the transformant according to Item 5.

### ADVANTAGES OF THE INVENTION

According to the present invention, in the production of a recombinant protein using a monocotyledon, the efficiency of the translation by a 5'UTR is improved, and efficient production of a recombinant protein becomes possible. According to the present invention, the same translation efficiency improving effect is exhibited even when the reporter gene is changed or the monocotyledon species is changed, and thus a recombinant protein can be produced with high versatility.

### BRIEF DESCRIPTION OF THE DRAWINGS

Fig. 1 shows a conceptual diagram of CAGE analysis.
Fig. 2 shows a conceptual diagram of polysome/CAGE analysis.
Fig. 3 shows an outline of expression vectors constructed to include a candidate 5'UTR.
Fig. 4 shows the results of a transient expression experiment in which a vector to which a candidate 5'UTR is linked is used.
Fig. 5 shows the results of a reproductive experiment for 5'UTRs having high translation capability.
Fig. 6 shows the results of verification of the translation capability in Arabidopsis thaliana of 5'UTRs confirmed to have high translation capability in rice.
Fig. 7 shows an outline of vectors for evaluating 5'UTR performance.
Fig. 8 shows performance evaluation of 5'UTRs when the reporter gene is changed.
Fig. 9 shows the results of a transient expression experiment in which a protoplast of rye is used.

### EMBODIMENTS OF THE INVENTION

Hereinafter, the present invention will be described in further detail. The abbreviations for amino acids, peptides, base sequences, nucleic acids and the like in the present specification are in accordance with the provision of IUPAC, IUB, "Guidelines for preparing specifications and the like including base sequences or amino acid sequences" (Edited by the Patent Office) and the symbols conventionally used in this field. In particular, DNA refers to deoxyribonucleic acid, RNA refers to ribonucleic acid, and mRNA refers to messenger RNA.

For molecular biological manipulation such as gene manipulation, a known method can be appropriately used. For example, unless otherwise specified, such molecular biological manipulation can be performed according to the method described in Molecular Cloning: A Laboratory Manual 3rd Edition (Cold Spring Harbor Laboratory Press) and the like.

### (1) DNA molecule encoding 5'UTR

The DNA molecule of the present invention is a DNA molecule encoding a 5'UTR, including a polynucleotide shown in any of (i) to (iii) below:
(i) a polynucleotide including a base sequence shown in any of SEQ ID NOs: 1 to 4,
(ii) a polynucleotide that includes a base sequence modified from the base sequence shown in any of SEQ ID NOs: 1 to 4 by substitution, deletion, or addition of one or several bases, and exhibits 5'UTR activity equivalent to that of the polynucleotide including the base sequence shown in any of SEQ ID NOs: 1 to 4, and
(iii) a polynucleotide that hybridizes under a stringent condition with a DNA fragment including a base sequence complementary to the base sequence shown in any of SEQ ID NOs: 1 to 4, and exhibits 5'UTR activity equivalent to that of the polynucleotide including the base sequence shown in any of SEQ ID NOs: 1 to 4.

Among the polynucleotides of (i) above, the polynucleotide including the base sequence shown in SEQ ID NO: 1 is a DNA molecule encoding a 5'UTR in Os04t0583600-01 gene of rice. Among the polynucleotides of (i) above, the polynucleotide including the base sequence shown in SEQ ID NO: 2 is a DNA molecule encoding a 5'UTR in Os08t0136800-01 gene of rice. Among the polynucleotides of (i) above, the polynucleotide including the base sequence shown in SEQ ID NO: 3 is a DNA molecule encoding a 5'UTR in Os02t0684500-00 gene of rice. Among the polynucleotides of (i) above, the polynucleotide including the base sequence shown in SEQ ID NO: 4 is a DNA molecule encoding a 5'UTR in Os06t0342200-01 gene of rice.

In the polynucleotides of (ii) above, the number of bases to be substituted, deleted, or added can be one or several, and specific examples thereof include 1 to 15, preferably 1 to 10, further preferably 1 to 8, particularly preferably 1 to 8, 1 to 7, 1 to 6, 1 to 5, 1 to 4, 1 to 3, 1 or 2, or 1.

In the polynucleotides of (iii) above, the "stringent condition" means a condition under which a pair of polynucleotides having high sequence similarity can specifically hybridize. A pair of polynucleotides having high sequence similarity means, for example, a pair of a polynucleotides having an identity of 80% or more, preferably 90% or more, further preferably 95%, particularly preferably 98% or more. The identity between the polynucleotides of a pair of polynucleotides can be calculated using Blast homology search software at default setting. Specific examples of the "stringent condition" include a condition in which hybridization is performed under a temperature condition of 42°C using 5 × SSC (83 mM NaCl, 83 mM sodium citrate).

In the polynucleotides of (ii) and (iii) above, "exhibiting 5'UTR activity equivalent to that of the polynucleotide including the base sequence shown in any of SEQ ID NOs: 1 to 4" means that the expression level of a recombinant protein when the recombinant protein is produced by a monocotyledon (including a monocotyledon cell) using the polynucleotide of (ii) as a 5'UTR is equivalent to the expression level of the recombinant protein when the polynucleotide including the base sequence shown in any of SEQ ID NOs: 1 to 4 is used as a 5'UTR. Specifically, when the expression level of a recombinant protein when the recombinant protein is produced using the polynucleotide including the base sequence shown in any of SEQ ID NOs: 1 to 4 as a 5'UTR is 100%, the expression level of the recombinant protein when the recombinant protein is produced using the polynucleotides of (ii) and (iii) above as a 5'UTR is 80% or more, preferably 85 to 120%, further preferably 90 to 120%, and particularly preferably 95 to 120%.

Though the polynucleotides of (i) above can be obtained from rice according to a known method, it can also be obtained by chemical synthesis. The polynucleotides of (ii) and (iii) above can be obtained by modifying the polynucleotides of (i) above using a known genetic engineering method, or can be obtained by chemical synthesis.

The polynucleotides of (i) to (iii) above are used by being linked to a 5'-terminal side of a polynucleotide encoding a recombinant protein as a 5'UTR in the production of a recombinant protein by a monocotyledon.

### (2) Nucleic acid construct including the DNA molecule

The nucleic acid construct of the present invention includes a polynucleotide encoding a foreign protein; and the polynucleotides of (i) to (iii) above linked to the polynucleotide encoding a foreign protein.

In the nucleic acid construct of the present invention, the polynucleotides of (i) to (iii) above function as 5'UTRs, and thus only need to be linked to a 5'-terminal side of the polynucleotide encoding a foreign protein.

In the nucleic acid construct of the present invention, the type of the foreign protein encoded is not particularly limited as long as the production thereof as a recombinant protein is required, and examples thereof include a protein having pharmacological activity. Specific examples thereof include medical proteins such as an enzyme, a transcription factor, a cytokine, a membrane-associated protein, various peptide hormones (for example, insulin, growth hormone, and somatostatin), a vaccine, and an antibody. In the nucleic acid construction of the present invention, a polynucleotide encoding a reporter protein such as GFP and luciferase, or a tag peptide such as His tag and FLAG (registered trademark) tag can be linked to the polynucleotide encoding a protein as needed.

In the nucleic acid construct of the present invention, known polynucleotides can be used as the polynucleotide encoding a foreign protein. The base sequence of such polynucleotides can be obtained from a database such as the sequence database GenBank managed by NCBI (National Center for Biotechnology Information). Based on such base sequence information, a polynucleotide encoding a protein can be isolated from various organisms by an ordinary method such as PCR. The polynucleotide is sold in the form of, for example, a cDNA library from sales companies, and can be purchased and used.

In the nucleic acid construct of the present invention, the origin of the foreign protein encoded is not particularly limited as long as the foreign protein encoded is a foreign protein homogeneous or heterologous to the host to which the nucleic acid construct is introduced. In the nucleic acid construct of the present invention, when the codon usage of the host to which the nucleic acid construct is introduced is known, the base sequence of the polynucleotide encoding a foreign protein can be changed to be matched to the codon usage suitable for the host.

### (3) Vector including the nucleic acid construct

The vector (expression vector) of the present invention can be obtained by linking the nucleic acid construct so that the nucleic acid construct can be expressed in the vector. More specifically, the vector of the present invention can be obtained by linking the nucleic acid construct to a vector having a promoter sequence immediately after the transcription start site of the promoter.

The vector to which the nucleic acid construct is inserted is not particularly limited as long as it can replicate in the host, and examples thereof include a plasmid vector, a cosmid vector, a viral vector, and an artificial chromosome vector (for example, YAC, BAC, and PAC). Of these, examples thereof preferably include a plasmid vector and a viral vector. In particular, from the viewpoint of expressing a recombinant protein in a monocotyledon (including a monocotyledon cell) further more efficiently, examples thereof include more preferably a plasmid derived from Agrobacterium, particularly preferably a plasmid derived from Agrobacterium and having T-DNA (Ti-plasmid).

In the present invention, a vector having a promoter sequence is used. A suitable promoter sequence can be appropriately selected and used according to the type of the host, and examples thereof include CaMV35S promoter, a promoter derived from cauliflower mosaic virus.

The vector to which the nucleic acid construct is inserted can include a gene that can be used as a selection marker such as a drug resistance gene.

As a vector to which the nucleic acid construct is inserted, a known vector, a vector commercially available from sales companies and the like can be used.

The nucleic acid construct can be incorporated and linked to a vector according to a known genetic engineering method. For example, the nucleic acid construct can be amplified by PCR using a primer having a restriction enzyme site added thereto, treated with a restriction enzyme, and linked and introduced to a vector treated with the restriction enzyme.

The vector of the present invention is one in which the nucleic acid construct is linked immediately after the transcription start site of a promoter. For example, in the cloning method in which a restriction enzyme is used, the restriction enzyme site is in the linking part between the promoter sequence and the nucleic acid construct. In such a case, for example, inverse PCR can be performed to remove the restriction enzyme site, and the obtained amplification product can be self-ligated to prepare a vector from which the restriction enzyme site in the linking part is removed. In this case, the primer set used for the inverse PCR is preferably designed to allow self-ligation of the PCR amplification product. For self-ligation, for example, a ligase can be used.

The "linking" the nucleic acid construct "immediately after the transcription start site" of the promoter sequence means that the nucleic acid construct and a promoter sequence are linked so that a transcription product in which 0, 1, 2, or 3 bases (preferably 0, 1, or 2 bases) transcribed from the promoter sequence is bound to the 5'-terminal (i.e., 5'UTR terminal) of the produced mRNA will be obtained when the polynucleotide encoding a protein in the nucleic acid construct is transcribed in the host. That is, in other words, linking is performed so that there will be no extra base sequence between the promoter sequence and the nucleic acid construct. Thus, a few bases (for example, 1, 2 or 3 bases) of the promoter sequence may be transcribed during gene expression even when the promoter sequence and the nucleic acid construct are directly linked. Vectors that cause such transcription are also included in the vector of the present invention.

### (4) Transformant including the vector

The transformant of the present invention can be obtained by introducing the vector of the present invention into a monocotyledon or a monocotyledon cell.

The type of the monocotyledon used as a host is not particularly limited, and examples thereof include rice, barley, wheat, corn, sorghum, purple false brome, sugarcane, and onion, preferably include rice, barley, wheat, and corn, and further preferably include corn and barley.

The type of the monocotyledon cell used as a host is also not particularly limited, and examples thereof include a rice-derived cell, a barley-derived cell, a wheat-derived cell, a corn-derived cell, a sorghum-derived cell, a purple false brome-derived cell, a sugarcane-derived cell, and an onion-derived cell, preferably include a rice-derived cell, a barley-derived cell, a wheat-derived cell, and a corn-derived cell, and further preferably include a rice-derived cell and a barley-derived cell. A protoplast derived from a monocotyledon cell is also included in a monocotyledon cell. A monocotyledon plant body obtained by culturing a transformed monocotyledon cell is also included in the transformant of the present invention.

When a tumor tissue, a shoot, a hairy root or the like is obtained as a result of transformation, it can be directly used for cell culture, tissue culture, or organ culture. It can be also regenerated into a plant body, for example, by administering a plant hormone such as auxin, cytokinin, gibberellin, abscisic acid, ethylene, and brassinolide at a suitable concentration by a conventionally known plant tissue culture method. A transformed plant body can be regenerated by using a transformed plant cell. As a regeneration method, a method in which callus-like transformed cells are transferred to a medium in which the type and concentration of the hormone are changed, and cultured to form an adventitious embryo to obtain a complete plant body is employed. Examples of the medium used include LS medium and MS medium.

The method of introducing the vector into a host is not particularly limited, and a suitable known method can be appropriately selected and used depending on the type of the host and the vector. Examples thereof include electroporation method, particle gun method, and a method in which Ti plasmid is used (for example, binary vector method and leaf disk method).

Whether or not the vector has been incorporated into the host can be confirmed by PCR method, Southern hybridization method, Northern hybridization method or the like. For example, DNAs are prepared from the transformant, a vector-specific primer is designed, and PCR is performed. Then, the amplification product is subjected to agarose gel electrophoresis, polyacrylamide gel electrophoresis, capillary electrophoresis or the like, and stained with ethidium bromide, SYBR Green solution or the like to detect the amplification product as one band, thereby transformation is confirmed. The amplification product can be also detected by performing PCR using a primer labeled in advance with a fluorescent dye or the like. Further, a method in which the amplification product is bound to a solid phase such as a microplate to confirm the amplification product by fluorescence or enzyme reaction can be also employed.

The transformant of the present invention is transformed with the vector, and thus transcription of the mRNA and translation of the foreign protein are performed from the polynucleotide encoding a foreign protein. As described above, by using the polynucleotides of (i) to (iii) above as a 5'UTR, efficient expression of a recombinant protein is possible in a monocotyledon or monocotyledon cell. Thus, a recombinant protein can be efficiently produced by culturing or cultivating the transformant of the present invention. The recombinant protein can be obtained by culturing or cultivating the transformant of the present invention, and then, for example, recovering and purifying the recombinant protein by a known method.

### EXAMPLES

Hereinafter, though the present invention will be specifically described, the present invention is not limited to the examples below. The materials used in the experiments are first described, and then the specific experimental contents and results are described.

### 1. Plant and cultured cell used

The following plant bodies and cultured cells were used in the following studies.

### 1-1. Rice cultured cell

Rice cultured cells (Oryza sativa cv. Nipponbare) were used for the following polysome/CAGE analysis. Culturing was performed under the condition of 30°C, dark period, and stirring speed of 90 rpm (EYELA, MULTI SHAKER, Tokyo), and 95 mL of R2S medium was placed in a 300 mL Erlenmeyer flask and used. Every week, 8 mL of cells that have reached stationary phase were transferred to 95 mL of new medium and subcultured.

Rice cultured cells (Oryza sativa L. C5924) Oc cells were used for the production of a protoplast. Culturing was performed under the condition of 30°C, dark period, and stirring speed of 90 rpm (EYELA, MULTI SHAKER, Tokyo), and 95 ml of R2S medium was placed in a 300 mL Erlenmeyer flask and used. Every week, old medium components were removed from 10 mL of cells that have reached stationary phase, then 20 mL of new R2S medium was added, and subculture was performed in a 100 mL flask.

### 1-2. Arabidopsis thaliana T-87 cultured cell

Arabidopsis thaliana cultured cells (Arabidopsis thaliana T87) used were those provided by Plant Development Bank, Gene Bank Office, RIKEN. Culturing was performed under the condition of 22°C, 18 hours of light period/6 hours of dark period, and stirring speed of 120 rpm (SLK-3-FS, NK system, Osaka), and 95 mL of modified LS medium (Nagata, T., Nemoto, Y., Hasezawa, S. (1992). Tobacco BY-2 cell line as the HeLa cell in the cell biology of higher plants. Int. Rev. Cytol. 132, 1-30.) was placed in a 300 mL Erlenmeyer flask and used. Every week, 4 mL of cells that have reached stationary phase was transferred to 95 mL of new medium and subcultured. When a transient expression experiment was performed, every week, 8 mL of cells that have reached stationary phase was transferred to 95 mL of new medium and subcultured.

### 2. Rye plant body

Seeds of rye (Secale cereal) were seeded on a 9 cm petri dish over which Kimwipe is spread, and then grown in a plant incubator LH-241/411RF(PID)T-S (25°C, 11 hours of light period, 13 hours of dark period).

### 3. Content and result of experiment

### 3-1. Polysome/CAGE analysis as evaluation method for translation state of mRNA

In general, the translation state of mRNAs is judged using the number of ribosomes bound to mRNAs as an index: translation is actively performed when a large number of ribosomes are bound to the mRNA (polysome), and translation is not performed when ribosomes are not bound (non-polysome) (Bailey-Serres, J., Sorenson, R., Juntawong, O. (2009). Getting the message across: cytoplasmic ribonucleoprotein complex. Trends Plant Sci. 14:443-453). Such a method is called polysome analysis, and the translation state of the mRNA can be evaluated at genome scale in combination with DNA microarray analysis.

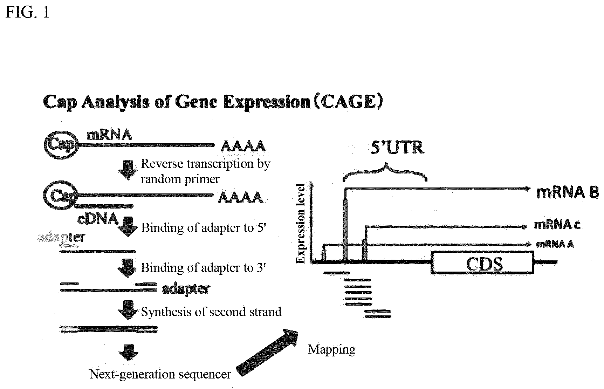

By the number of bound ribosomes, mRNAs were fractionated, and then Cap Analysis of Gene Expression (CAGE) analysis was performed. CAGE analysis is a method capable of identifying a transcription start site (TSS) and quantifying the transcription product for each TSS. Fig. 1 shows a conceptual diagram of CAGE analysis. As shown in Fig. 1, a complementary strand cDNA is synthesized from total RNA extracted from cells and tissues using a random primer, and the 5'-terminal of the cDNA is screened by cap-trapping method. An adapter (including a recognition site necessary for cloning, a short specific base sequence as a mark, and an endonuclease recognition site) is bound to the 5'-terminal of the single-stranded cDNA obtained by removing the RNA strand. The second strand cDNA is synthesized, and then 20 bases (Tag sequence) from the 5'-terminal are cut out with a restriction enzyme. Two linkers (including another restriction enzyme recognition site) are bound to the 3'side of the cut out Tag sequence. The cDNA tag is purified, the recognition sites on the linkers at both ends are cut out with a restriction enzyme, and the Tag sequence interposed between the linkers is cut out. The DNA sequence of the Tag thus obtained is determined with a next-generation sequencer and mapped on the genome. Thereby, the transcription start site of various mRNAs can be identified, that is, the 5'UTR can be estimated. Further, the mRNA can be quantified. In Fig. 1, three types of variants (mRNA A, mRNA B, and mRNA C) having different transcription start sites are illustrated, and the 5'UTR region of the mRNA B is shown as a representative.

### 3-2. PR value as index of translation state

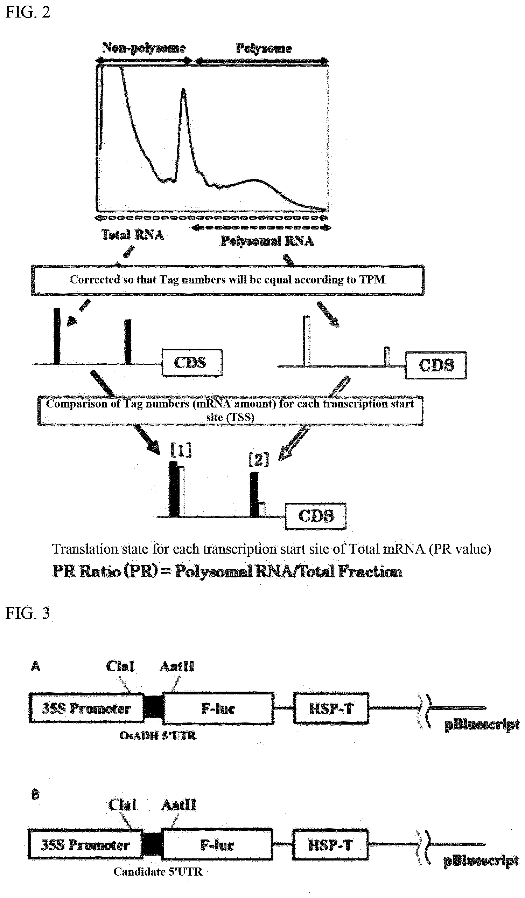

In the present invention, the degree of polysome formation (PR value) in the mRNA transcribed from each TSS of each gene was evaluated by performing polysome/CAGE analysis. Fig. 2 shows the conceptual diagram. As shown in Fig. 2, Polysomal RNA fraction (that is, mRNAs of a polysome to which two or more ribosomes are bound) and Total RNA (that is, all mRNAs including nonpolysomes and polysomes) according to polysome analysis were first subjected to CAGE analysis. The Tag number of the Polysomal RNA fraction was corrected so that the Tag number of the Total RNA fraction and the Tag number of the Polysomal RNA fraction in the data obtained in CAGE analysis would be equal. The Tag numbers (mRNA amount) for each transcription start site (TSS) were compared to calculate a Polysome Ratio value (PR value), the ratio of the Tag number of the Polysomal RNA fraction to the Tag number of the Total RNA fraction. In the example of Fig. 2, [1] and [2] are given in descending order of the PR value.

The PR (Polysome Ratio) value is a numerical value of the ratio of the amount of each mRNA in the polysomal fraction to the amount of total mRNA, and is one of the indexes of the translation state. It is expected that the higher the PR value of the mRNA is, the more actively the mRNA is translated. Rice cultured cells were used for the analysis. Rice cultured cells have a track record for practical use for the actual substance production in a monocotyledon, the contaminants are less than that of the plant body, and the translation state can be accurately evaluated. It is known that the translation state of rice cultured cells is greatly suppressed by environmental stress and the like, and the translation state of the transgene is also presumably suppressed similarly. Thus, the present inventor aimed to search for a 5'UTR sequence that performs active translation even under a wide range of conditions such as environmental stress by analyzing, in addition to cells under normal conditions, cells under heat stress, which is known to cause extremely strong translation suppression.

### 3-3. Polysome analysis by sucrose density-gradient centrifugation

The cells on the 3rd day of culture (normal conditions, 30°C, 90 rpm) and
the cells heat-treated in a water bath (heat conditions, 41°C, 15 min, 90 rpm) were each collected and rapidly cooled with liquid nitrogen. The rapidly cooled samples were crushed using a mortar, and were dispensed into a 2 ml tube in 300 mg each. Polysome fractionation by sucrose density-gradient centrifugation was basically performed according to the method of Davies et al. except some modifications (Davies E, Abe S. Chapter 15 Methods for Isolation and Analysis of Polyribosomes. Methods in Cell Biology, Volume 50, 1995, Pages 209-222). To the crushed sample powder, Extraction Buffer (200 mM Tris-HCl, pH 8.5, 50 mM KCl, 25 mM MgCl₂, 2 mM EGTA, 100 µg/ml heparin, 100 µg/ml cycloheximide, 2% polyoxyethylene 10-tridecyl ether, 1% sodium deoxycholate) about 4 times (w/v) the crushed sample powder was added to gently suspend the crushed sample powder. The suspension was centrifuged (14,000 × g, 15 min, 4°C) to remove the cell debris and further centrifuged (14,000 × g, 10 min, 4°C). The supernatant was used as a crude RNA extract. The crude extract was adjusted to an RNA concentration of 333 ng/µl with Extraction Buffer, and 300 µL of the crude extract was overlaid on 4.85 mL of 26.25 to 71.25% sucrose density-gradient solution prepared in advance (sucrose, 200 mM Tris-HCl, 200 mM KCl, 200 mM MgCl₂) to perform ultracentrifugation (SW55Ti rotor, 55,000 rpm, 50 min, 4°C, brake-off) (Optima, Beckman Coulter, California, USA). Aspiration was performed from the top of the sucrose density-gradient at a rate of about 1 mL/min with Piston gradient fractionator (BioComp, Churchill Row, Canada), and at the same time the absorbance at 254 nm was recorded with BIO-MINI UV MONITOR AC-5200 (ATTO, Tokyo, Japan).

### 3-4. Extraction of RNA for CAGE analysis

From the sucrose density-gradient solution after ultracentrifugation, about 2 mL of the fraction containing the polysome-forming mRNA and about 4 mL of the total fraction were each collected in a centrifuge tube to which 8 M guanidine hydrochloride had been added in advance so that the final concentration would be 5.5 M. To each centrifuge tube, 100% ethanol in an amount equal to the amount of the mixture was added, the mixture was cooled at -20°C overnight, and then subjected to centrifugation (40,000 × rpm, 45 min, 4°C). The obtained pellet was washed once with 85% ethanol (40,000 x rpm, 30 min, 4°C), then the pellet was dissolved with 1 mL of buffer RLT included in RNeasy kit (Qiagen, Hilden, Germany), and thereafter the RNA was purified using the RNeasy kit according to the attached protocol to obtain Polysomal RNA fraction and Total RNA fraction.

### 3-5. Production of CAGE library and analysis by next-generation sequencer

The extracted RNA was used to produce a Cap Analysis of Gene Expression (CAGE) library. The method for producing the CAGE library was in accordance with the method for producing the nAnT-iCAGE library of Murata et al. (Murata, M., Nishiyori-Sueki, H., Kojima-Ishiyama, M., Carninci, P., Hayashizaki, Y., Itoh, M. (2014). Detecting expressed genes using CAGE. Methods Mol. Biol. 1164: 67-85.). The outline is as follows. A complementary strand cDNA was synthesized using N15 random primer, and the 5'-terminal of the cDNA was screened by cap-trapping method. Then, linkers were bound to the 5'-terminal and 3'-terminal sides of the single-stranded cDNA obtained by removing the RNA strand using RNase I to obtain a CAGE library. Sequencing was performed according to the attached protocol using Illumina(R)HiSeq 2500 in single read in which the sequence primer recognition site present in the linker bound to the 5'-terminal is used. The analysis was performed for each RNA sample using two repetition samples independent from the time of cell collection (that is, 8 samples in total).

### 3-6. Data processing and mapping

The CAGE linker sequence was removed from each Tag in the raw data obtained by sequencing. Then, a Tag having N that indicates incorrect reading in the sequence, a Tag derived from a contaminant rRNA, and a Tag not having G derived from Cap at the 5'-terminal were removed to extract a Tag that is presumably derived from the 5'-terminal of the mRNA having Cap. Then, mapping was performed based on the information of Os-Nipponbare-Reference-IRGSP-1.0 (http://rapdb.dna.affrc.go.jp/). At this time, the Tag having low mapping quality that was mapped at multiple positions and the Tag that may have a mismatch at the 5'-terminal and have a mismatched terminal position were removed. The positions on the genome where Tags were mapped were identified, and Tags at each position was counted. Tags were counted only in the position in the genome where a Tag is present in both two independently obtained samples and reproducibility is confirmed, and the Tag number was converted to Tag per million (TPM) value. Then, the Tag that is present between 500 nt upstream of the 5'-terminal and AUG of CDS (Coding DNA Sequence) of any gene registered in Os-Nipponbare-Reference-IRGSP-1.0, and is consistent in the strand direction was annotated as indication of a transcription start site (TSS) of the gene. The Tag that was mapped to a region having no gene or to a CDS region and was not annotated for a gene was removed. The TPM value at the finally obtained Tag position was taken as the TPM_TSS value that indicates the amount of the mRNA derived from the TSS. Subsequently, the proportion of the mRNA transcribed from each TSS that forms a polysome was calculated as the PR_TSS value by dividing the TPM_TSS value of the Polysomal RNA fraction by the value of the Total RNA fraction.

### 3-7. Production of translation state ranking

By polysome/CAGE analysis in rice, very large-scale translation state of each TSS level (PR_TSS value) and sequence information of 5'UTRs deduced from the TSS were obtained. Using these data sets, a ranking was produced to select a 5'UTR of a mRNA having good translation state as a candidate. In the ranking, to obtain a 5'UTR candidate that actively performs translation under various conditions, the values obtained by converting the PR_TSS values under normal conditions and heat conditions to Z-scores and averaging the Z-scores (Mean of Normarized PR value) were used. Conversion to Z-score is one of correction methods, and is a useful method when the bias of importance between datasets having widely different distributions is reduced and averaged like this time. In the production of a ranking, the 5'UTR sequence having uAUG and an intron was excluded because it may not be capable of correctly translating the target gene when used as a translation enhancer. In the method of polysome analysis,
as the CDS length becomes longer, the probability of presence of a ribosome in the process of translation becomes higher, and thus, the PR value tends to increase. Thus, when the 5'UTR has a long CDS, which of the 5'UTR and the CDS length is responsible for the high PR_TSS value cannot be determined. Thus, the 5'UTR having a CDS length not less than the median (864 nt) was removed.

Finally, a ranking of mRNAs derived from 10,099 TSSs derived from 2907 genes common to both conditions was produced, and from them, the top 15 5'UTRs were selected as candidates (Table 1). In Table 1, Os-Nipponbare-Reference-IRGSP-1.0 name (Gene Name), 5'UTR length, PR_TSS value of cells under normal conditions (PR_Con), PR_TSS value of cells under heat conditions (PR_Heat), Mean of PR_TSS value of cells under normal conditions and heat conditions after conversion to Z-Score (Mean of Normarized PR), PR_TSS value ranking of cells under normal conditions (PR_Rank_Con), and PR_TSS value ranking of cells under heat conditions (PR_Rank_Heat) are shown. Further, the sequence information of each 5'UTR is shown in Table 2.

**[Table 1]**

| Rank | Gene Name | 5'UTR length | PR_Con | PR_Heat | Mean of Normarized PR | PR_Rank_Con | PR_Rank_Heat |
|---|---|---|---|---|---|---|---|
| 1 | Os10t0530900-01 00076 | 76 | 1.67 | 2.11 | 2.81 | 15 | 5 |
| 2 | Os04t0556300-01 00118 | 118 | 1.64 | 2.02 | 2.62 | 27 | 22 |
| 3 | Os03t0180400-01 00112 | 112 | 1.67 | 1.97 | 2.61 | 20 | 32 |
| 4 | Os11t0592100-01 00063 | 63 | 1.66 | 1.98 | 2.61 | 21 | 29 |
| 5 | 0s05t0119200-01 00064 | 64 | 1.7 | 1.85 | 2.51 | 7 | 68 |
| 6 | Os01t0303800-01 00106 | 106 | 1.53 | 2.07 | 2.47 | 112 | 11 |
| 7 | Os06t0147400-01 00074 | 74 | 1.59 | 1.97 | 2.45 | 54 | 34 |
| 8 | Os02t0684500-00 00094 | 94 | 1.49 | 2.11 | 2.44 | 176 | 6 |
| 9 | Os08t0117400-01 00160 | 160 | 1.7 | 1.77 | 2.42 | 6 | 129 |
| 10 | 0s01t0559000-01 00115 | 115 | 1.74 | 1.68 | 2.38 | 4 | 218 |
| 11 | Os07t0230700-01 00066 | 66 | 1.67 | 1.78 | 2.36 | 19 | 116 |
| 12 | Os08t0136800-01 00129 | 129 | 1.31 | 2.31 | 2.34 | 1085 | 2 |
| 13 | Os04t0583600-01 00068 | 68 | 1.43 | 2.08 | 2.3 | 313 | 10 |
| 14 | Os06t0342200-01 00113 | 113 | 1.45 | 2.05 | 2.29 | 268 | 15 |
| 15 | Os08t0136800-01 00116 | 116 | 1.55 | 1.89 | 2.27 | 89 | 49 |

**[Table 2]**

| Rank | Gene Name | Sequence |
|---|---|---|
| 1 | Os10t0530900-01_00076 | ATAAGCAAACCAAACAAAAAAACCTCACCATCATCGACCCACTTGTT CCTAGCGATCTAGAACGAGAGACTTGGCA |
| 2 | Os04t0556300-01_00118 | ACGTCTCGTCTCTGTCTCTCGGCCCACCGCTTCCATCGTTCGTCCTC GTCTCCACGCTACCGTTTCGCAACCACCCGCTTCCTCCTCCGGAGAC CGCTCGGCCGCCGCTCGCTCCAGC |
| 3 | Os03t0180400-01_00112 | ATTTTTGGTTTATTTTGCCTCCCCAACCAAGCACAAACACAAGCCTCG CCGTATTCTCTCGGAGCAAAGCGAACGCGAAACCCCACCCGCGGCG GCGAGAAATCCGGCGACG |
| 4 | Os11t0592100-01_00063 | ATTTCACTAGCATCTAGCAAGTGCACAACACATCTATAAATCGTAGT AACCACCTTAGGTGAG |
| 5 | Os05t0119200-01_00064 | AGTTTTCTCCTCTGATTTTCCACCACAAGATCTCTAGTTTCTTTGATT TCTTGATCAGACGACG |
| 6 | Os01t0303800-01_00106 | ATCTCTCCACTTCTCCAACCAATTGCCCGATTCATCGATCTCTCATCG TTCGTTCGCTCTAGCTAGGTTGAAGACTCGGGAGTCTAGCTAGCTGC TGTCGAGAGTG |
| 7 | Os06t0147400-01_00074 | GTTTTGAGTGCTGCGAACGCAGAGACTAGACTTTTGACCACCGCGTG AGTCCAACCCTTCACGGCGAGGGAACC |
| 8 | Os02t0684500-00_00094 | ACCAAGAGCCACAGCGATTTCAGATCTCCCCAAGTAGTAGACTTCTC TCTTCTTCCATCTCGGATTCGTCTCGGTCTCGTCTCGGTCTCCGGCG (SEQ ID NO: 3) |
| 9 | Os08t0117400-01_00160 | ACTTTGTTCCCCACCACAGCAAGCGCAACGCGCAAAGAGGCAGACG CGCAGCTGAGGAGGAGAAGCGCACGCAGCCACCGCGAGATCTCCTC ACCTCCATCTCCATCTCCATCTCCATCTCCGGCGGCGAGATCCCCAC CGAATCGGATCGGATCGGATC |
| 10 | Os01t0559000-01_00115 | AGGGAGTGAAGAGCGAATCAAACACCACCACCATCGCCAGCGTCATT CAGATTTCAGAGGGAGGGAGGGAGGGAGAGAGAAAGGAAGGGGAA GTGGGGGAGAAGGAGGGGAGAGG |
| 11 | Os07t0230700-01_00066 | AATTAACCTGACGCCACCACCTCTCTTTCCCTCTCTCTCTTCCATTCA CCGACAATCTAACTAAAA |
| 12 | Os08t0136800-01_00129 | GAACACACAACCCAGCTATAGCCACCTACATCTGCTGATCGACATCG ATCGATCTCTACTACTCCAGTAGCTAGCAAGAATTGAATCTAGGTGA AGTGAGAGTGTGAGAGTACATAATTGGCGCCATCC (SEQ ID NO: 2) |
| 13 | Os04t0583600-01_00068 | ACAAGGCTTCTTCCACATCTCCACCCAAATTCCAAATCTCTCGGTGAT CGGAGCGGTTAGCTGCGGCG (SEQ ID NO: 1) |
| 14 | Os06t0342200-01_00113 | CAGAAAAACCGCATCCCGCAACTCCACCGCTCCCAATCTCGATCGAA ACGCGAGGGTTTCGCATCTCCGCCGACGCCTAGAGGAAGAAGGGAT CCGCCGCCGGTAGCCAAGCC (SEQ ID NO: 4) |
| 15 | Os08t0136800-01_00116 | AGCTATAGCCACCTACATCTGCTGATCGACATCGATCGATCTCTACT ACTCCAGTAGCTAGCAAGAATTGAATCTAGGTGAAGTGAGAGTGTGA GAGTACATAATTGGCGCCATCC |

### 3-8. Evaluation of translation capability of candidate 5'UTR by DNA transient expression experiment

As a basic vector used for transient expression experiment, an expression vector (pBluescript) including OsADH 5'UTR of Oryza sativa Indica Group (long-grained rice) under the control of the CaMV35S promoter, Firefly luciferase gene (F-luc), and a terminator (derived from Heat Shock Protein 18.2 gene) was used. The restriction enzyme sites AatII and ClaI present in this vector were treated with restriction enzymes to linearize the vector DNA. Then, to amplify the candidate 5'UTR gene-specifically, a primer was designed based on the information of Os-Nipponbare-Reference-IRGSP-1.0 (http://rapdb.dna.affrc.go.jp/). AatII and ClaI, which are restriction enzyme sites in the basic vector, were added to the designed primers. PCR was performed using rice genomic DNA as a template to amplify the target fragment. The linearized basic vector was linked to 15 types of the DNA fragment of the candidate 5'UTR according to In-Fusion cloning (TaKaRa) protocol (Fig. 3). As the R-luc gene for correcting the transfection efficiency, pBluescriptII KS+ having an expression cassette composed of 35S promoter, R-luc gene, and HSP terminator was used.

DNA (1 µg of F-luc, 0.4 µg of R-luc, the total volume of around 10 µl) was cointroduced to a protoplast prepared from rice cultured cells (Oc cells) by Polyethylene glycol (PEG) method (Kovtun, Y., Chiu, W. L., Tena, G., Sheen, J. (2000). Functional analysis of oxidative stress-activated mitogen-activated protein kinase cascade in plants. Proc. Natl. Acad. Sci. USA. 6: 2940-2945.), and the resulting product was allowed to stand at 30°C for 16 hours, and then centrifuged to remove the supernatant. Then, the resulting product was frozen with liquid nitrogen and stored at -80°C. Then, the cells were lysed using passive lysis buffer (Promega Wisconsin, USA), the F-luc activity value and R-luc activity value in the lysate were measured with Dual-luciferase reporter assay system (Promega) and a plate reader (TriStar LB 941, BERTHOLD TECHNOLOGIES), and the relative activity value (F-luc activity value/R-luc activity value) was calculated.

For comparison, OsADH 5'UTR, which has been reported to have high translation capability (Sugio, T., Satoh, J., Matsuura, H., Shinmyo, A., Kato, K. (2008). The 5'-untranslated region of The Oryza sativa alcohol dehydrogenase gene functions as a translational enhancer in monocotyledonous plant cells. J. Biosci. Bioeng. 105: 300-302.), was also evaluated in the same manner, and the relative activity value of each candidate 5'UTR (Relative F/R activity) was calculated as a relative value when the relative activity value of OsADH 5'UTR is 1 (relative activity value of candidate 5'UTR/relative activity of OsADH 5'UTR).

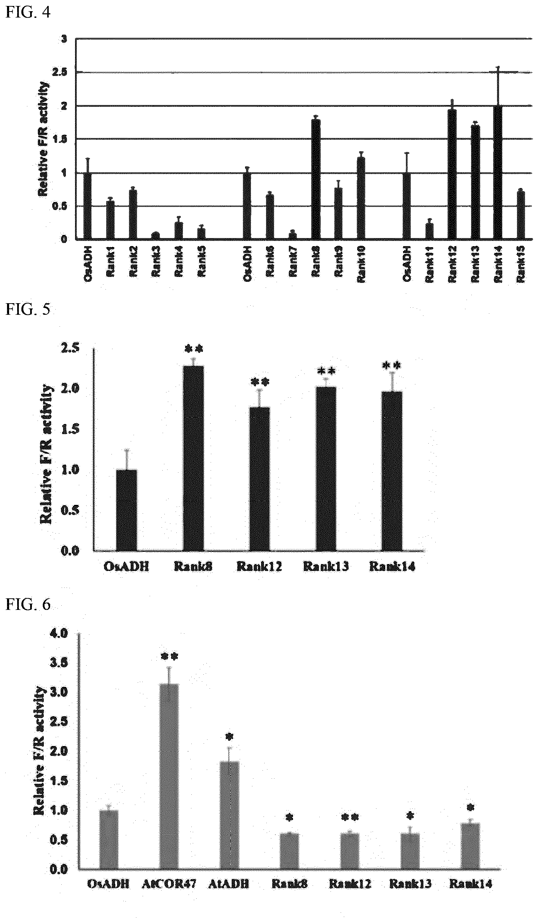

The relative activity value (Relative F/R activity) for each candidate is shown in Fig. 4 together with OsADH 5'UTR. In Fig. 4, the mean and the standard error of the relative activity values based on the F-luc activity value and the R-luc activity value measured in each experiment independently performed three times from the introduction of the vector into protoplasts are shown. Further, the difference from the F-luc expression vector linked to OsADH 5'UTR was evaluated by Welch's t-test. In the figure, * indicates p < 0.05, and ** indicates p < 0.01. As shown in Fig. 4, it was confirmed that 5'UTRs that exhibit a translation capability lower than the translation capability of OsADH 5'UTR for comparison and 5'UTRs that exhibit a translation capability higher than the translation capability of OsADH 5'UTR for comparison are mixed in 15 candidate 5'UTRs selected as having high translation capability. In particular, the difference in translation capability between Rank12 and Rank15, which are different 5'UTR variants derived from the same gene, was more than double.

From the 15 candidates, Rank8, Rank12, Rank13, and Rank14 that have translation capability significantly exceeding that of OsADH 5'UTR for comparison were selected, and a transient expression experiment was performed again to confirm the reproducibility. The results are shown in Fig. 5 in the same manner. In Fig. 5 also, the mean and the standard error of the relative activity values based on the F-luc activity value and the R-luc activity value measured in each experiment independently performed three times from the introduction of the vector into protoplasts are shown. Further, the difference from the F-luc expression vector linked to OsADH 5'UTR was evaluated by Welch's t-test. In the figure, * indicates p < 0.05, and ** indicates p < 0.01.

### 3-9. Performance evaluation of candidate 5'UTR sequence by transient expression experiment in which protoplast derived from Arabidopsis thaliana cultured cell (Arabidopsis thaliana T87) is used

Each expression vector to which the 5'UTR of Rank8, Rank12, Rank13, and Rank14 that showed relative activity value exceeding the translation capability of OsADH 5'UTR has been incorporated was introduced into a protoplast of Arabidopsis thaliana, a dicotyledon, and F-luc, a reporter, was transiently expressed to measure the relative activity value (F-luc activity value/R-luc activity value). For comparison, a relative activity value (F-luc activity value/R-luc activity value) was calculated from the R-luc activity value and the F-luc activity value in the same manner using a vector to which AtCOR47 5'UTR or AtADH 5'UTR having high translation capability in a dicotyledon is linked. Each calculated relative activity value (Relative F/R activity) was calculated as the relative value when the relative activity value of OsADH 5'UTR is 1 (relative activity value of candidate 5'UTR /relative activity value of OsADH 5'UTR).

The relative activity values (Relative F/R activity) for Rank8, Rank12, Rank13, Rank14, and the comparison 5'UTRs are shown in Fig. 6 together with OsADH 5'UTR. In Fig. 6, the mean and the standard error of the relative activity values based on the F-luc activity value and the R-luc activity value measured in each experiment independently performed three times from the introduction of the vector into protoplasts are shown. Further, the difference from the F-luc expression vector linked to OsADH 5'UTR was evaluated by Welch's t-test. In the figure, * indicates p<0.05, and ** indicates p<0.01.

As shown in Fig. 6, the F-luc expression levels of the 5'UTRs of Rank8, Rank12, Rank13, and Rank14 were significantly lower than those of AtADH 5'UTR and AtCOR47 5'UTR. From this, it was shown that the 5'UTR sequences having high translation capability in a monocotyledon cannot exhibit high translation capability in a dicotyledon. That is, it was shown that the excellent improving effect of translation capability of 5'UTRs of Rank8, Rank12, Rank13, and Rank14, which exceeds the translation capability of OsADH 5'UTR, is monocotyledon-specifically exhibited.

### 3-10. Performance evaluation of 5'UTR when reporter gene is changed

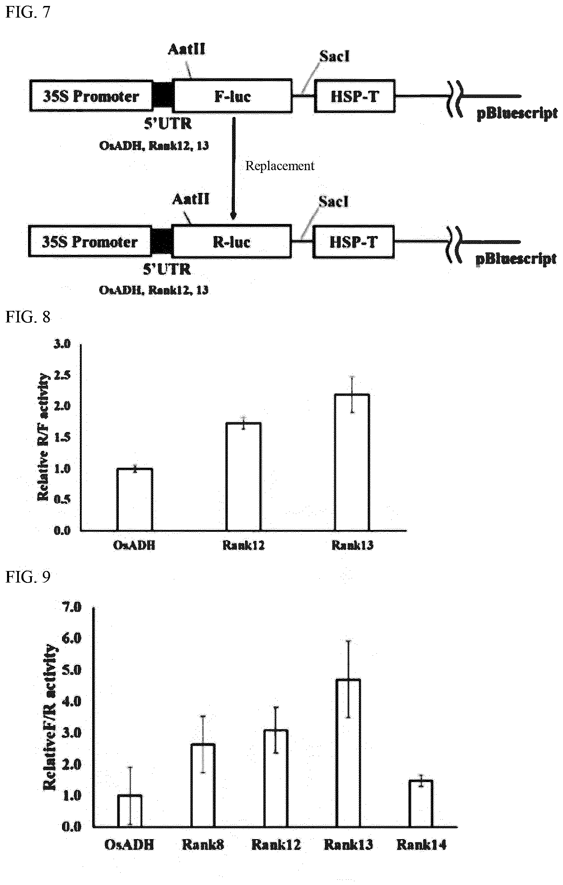

Influence of the replacement of the gene region on the high translation capability of 5'UTR was examined, considering the fact that the target genes are diverse when the transgene is actually expressed. A vector was first constructed in which the F-luc reporter gene was replaced with R-luc reporter gene. Each 5'UTR of OsADH, Rank12, and Rank13 was linked to this vector (Fig. 7). OsADH 5'UTR was used for comparison. Each R-luc expression vector was introduced into a protoplast of rice cultured cells (Oc cells) to transiently express the reporter R-luc. To correct the introduction efficiency into a protoplast, the F-luc expression vector to which OsADH 5'UTR has been inserted was also cointroduced, and the R-luc activity value relative to the F-luc activity value (R-luc activity value/F-luc activity value) was calculated. Each calculated relative activity value (Relative R/F activity) was calculated as the relative value when the relative activity value of OsADH 5'UTR is 1 (relative activity value of candidate 5'UTR /relative activity value of OsADH 5'UTR).

The relative activity values (Relative R/F activity) for Rank12 and Rank13 are shown in Fig. 8 together with OsADH 5'UTR. In Fig. 8, the mean and the standard error of the relative activity values based on the R-luc activity value and the F-luc activity value measured in each experiment independently performed three times from the introduction of the vector into protoplasts are shown. Further, the difference from the R-luc expression vector linked to OsADH 5'UTR was evaluated by Welch's t-test. In the figure, * indicates p<0.05, and ** indicates p<0.01.

As shown in Fig. 8, even when R-luc was used as a reporter gene (that is, even when the gene region was replaced), both the 5'UTR of Rank12 and the 5'UTR of Rank13 exhibited high translation capability compared to OsADH 5'UTR.

### 3-11. Performance evaluation of 5'UTR in transient expression experiment in which rye protoplast is used

Considering that the actual production of useful substances is performed in hosts other than rice, the fact that a 5'UTR having a high translation capability can be used universally across plant species was verified by performing a transient expression experiment using a protoplast derived from rye, also a monocotyledon, Poaceae. Each of Rank8, Rank12, Rank13, and Rank14 that exhibit relative activity value exceeding the translation capability of 5'UTR of OsADH was introduced into a rye protoplast, and the reporter gene F-luc was transiently expressed. To correct the introduction efficiency into a protoplast, the R-luc expression vector to which OsADH 5'UTR has been inserted was also cointroduced, and the F-luc activity value relative to the R-luc activity value (F-luc activity value/R-luc activity value) was calculated. Each calculated relative activity value (Relative F/R activity) was calculated as the relative value when the relative activity value of OsADH 5'UTR is 1 (relative activity value of candidate 5'UTR /relative activity value of OsADH 5'UTR).

The relative activity values (Relative F/R activity) for Rank8, Rank12, Rank13, and Rank14 are shown in Fig. 9 together with OsADH 5'UTR. In Fig. 9, the mean and the standard error of the relative activity values based on the F-luc activity value and the R-luc activity value measured in each experiment independently performed three times from the introduction of the vector into protoplasts are shown. Further, the difference from the F-luc expression vector linked to OsADH 5'UTR was evaluated by Welch's t-test. In the figure, * indicates p<0.05.

As shown in Fig. 9, Rank8, Rank12, Rank13, and Rank14 had activity values exceeding that of OsADH even in rye. That is, Rank8, Rank12, Rank13, and Rank14 were shown to be 5'UTR sequences that exhibit high translation capability even in rye.

## Claims

1. A DNA molecule encoding a 5'UTR, having:
a polynucleotide shown in any of (i) to (iii) below:
(i) a polynucleotide consisting of a base sequence shown in any of SEQ ID NOs: 1 to 4,
(ii) a polynucleotide that consists of a base sequence modified from the base sequence shown in any of SEQ ID NOs: 1 to 4 by substitution, deletion, or addition of one or several bases, and exhibits 5'UTR activity equivalent to that of the polynucleotide consisting of the base sequence shown in any of SEQ ID NOs: 1 to 4, and
(iii) a polynucleotide that hybridizes under a stringent condition with a DNA fragment consisting of a base sequence complementary to the base sequence shown in any of SEQ ID NOs: 1 to 4, and exhibits 5'UTR activity equivalent to that of the polynucleotide consisting of the base sequence shown in any of SEQ ID NOs: 1 to 4.

2. A nucleic acid construct, comprising:
a polynucleotide encoding a foreign protein; and
the DNA molecule according to claim 1 linked to a 5'-terminal side of the polynucleotide encoding a foreign protein.

3. A vector, comprising:
the nucleic acid construct according to claim 2.

4. A method for producing a transformant, comprising the step of:
introducing the vector according to claim 3 into a monocotyledon or a monocotyledon cell.

5. A transformant obtained by transforming a monocotyledon or a monocotyledon cell with the vector according to claim 3.

6. A method for producing a recombinant protein, comprising the step of:
culturing or cultivating the transformant according to claim 5.
